# EUROPEAN PATENT SPECIFICATION

(11) **EP 3 146 903 B1**
(45) Date of publication and mention of the grant of the patent: **08.05.2019**
(21) Application number: 15186551.6
(22) Date of filing: 23.09.2015
(51) Int. Cl.: A61B 17/00, A61B 17/12

(54) **OCCLUSION DEVICE FOR CLOSING AN APICAL HOLE IN THE HEART WALL**
OKKLUSIONSVORRICHTUNG ZUM VERSCHLIESSEN EINER APIKALEN ÖFFNUNG IN DER HERZWAND
DISPOSITIF D'OCCLUSION POUR FERMER UN TROU APICAL DANS LA PAROI CARDIAQUE

(43) Date of publication of application: 29.03.2017
(73) Proprietor: Lepu Medical Technology (Beijing) Co., Ltd., Beijing 102200 (CN)
(72) Inventor: Ferrari, Enrico, 6900 Lugano (CH); Sui, Ziye, Bolsward (NL); Yang, Yongsen, Shanghai (CN)
(74) Representative: Prinz & Partner mbB

(56) References cited:
- EP-A1- 2 014 240
- US-A1- 2007 118 176
- US-A1- 2009 062 841
- US-A1- 2009 099 647

## Description

The present invention relates to an occlusion device for use in heart surgery procedures for the closure of punctures made in the cardiac wall.

The apex of the heart refers to a lower portion of the heart, usually at the bottom part of the left ventricle. The thickness of the apex wall varies with each individual and may have a thickness of up to 18 mm.

In minimally invasive cardiac surgery, such as heart valve replacement or left ventricular bypass, the delivery device must be passed into the interior of the heart via an artificial hole created in the heart wall, particularly in the apex region. Specifically, transapical aortic valve replacement (TAVR) surgery has developed rapidly in recent years and there is still no completely satisfactory way of closing the puncture artificially created for TAVR in the heart wall.

### Description of the Related Art

Apical closure devices and procedures have been advanced over the past years since the introduction of TAVR surgery, as illustrated for example in the review article "Left Ventricular Apical Access and Closure for TAVR", by P. Vallabhajosyula and W. Y. Szeto, in Cardiac Interventions Today March/April 2013, page 45. The conventional closing procedures include purse-string suturing or a mattress suturing technique.

A known closure device of Apica Cardiovascular Ltd. (ACS system) consists of a spiral coil designed to be rotated into the myocardium tissue about the apical hole. A closure cap is provided for sealing the coil, which provides a working port for future interventions if needed. However, it is still necessary to penetrate cardio tissue surrounding the apical hole and there is some risk of the closure cap being dislodged from the coil.

US Patent No. 2012/0253386 A1 discloses another closure device comprising a main cylindrical body which can be deployed in a delivery device. Once deployed, prongs extend away from the axis of the main body and serve as anchors into the tissue surrounding the apical hole. The main body can then be collapsed to facilitate closure of the apical puncture.

Another closure system is disclosed in US 2012/0016411 A1. The closure apparatus comprises a tissue attachment portion arranged on the forward and rearward end of a cylindrical main body. The attachment portion includes hooks at both the distal end and proximal end of the cylindrical main body, which engage in the myocardium tissue around the hole for securement of the device. The cylindrical main body collapses toward the center of the hole and a sealing material is disposed within the center of the device to promote a sealing effect.

US 2009/0062841 A1 discloses a double-disk occlude for PDA treatment. The device in its relaxed, unstretched condition has two disks aligned in spaced relation and linked together by a central cylindrical segment. The length of the central segment may approximate the thickness of the atrial septum. The two disks may have an outer diameter sufficiently larger than the cavity opening to prevent dislodgement of the device. The larger diameter portions of the disks are arranged to face one another.

All of the above methods and devices for closure of the apical hole have the common disadvantage of the requiring anchors and/or sutures which puncture the myocardium tissue surrounding the hole.

The object of the present invention is to provide an improved apical occlusion device which requires little or no suturing or penetration of the myocardium and which is conveniently applied with a standard delivery device through the lumen of a catheter.

### Disclosure of the Invention

According to the present invention an occlusion device is provided as defined in claim 1. The device comprises a first disk having a first enlarged diameter flange portion adapted to be placed proximate an exterior end of an apical hole in a patient's heart wall and a first shoulder portion adapted to be placed within the apical hole near the exterior end.

A second disk is provided having a second enlarged diameter flange portion adapted to be placed proximate an interior end of the apical hole in the patient's heart wall and a second shoulder portion adapted to be placed within the apical hole near the interior end. A central waist portion extends between the first and second disks along a center axis of the device, which is adapted to extend through the apical hole and connect the first and second disks, while urging the disks toward one another when in the mounted condition.

The occlusion device is made of metal wire mesh comprising a plurality of metal strands made of a shape memory alloy material. The wires are preferably made of a nickel-titanium alloy.

In accordance with the present invention, the first and second shoulder portions have respective inner faces which lie opposite one another in the direction of a center axis of the device, where each inner face is inwardly concaved to form a recess in the relaxed condition of the device. The central waist portion interconnects the inner faces of the first and second shoulder portions, both in the relaxed condition of the device and in the expanded condition when the device is mounted in the apical hole. When mounted, the inner faces are pulled axially apart and the recessed faces take on a flatter shape, while the first and second shoulder portions become separated. In this expanded, mounted condition, the inner flatter faces are separated axially from one another and lie substantially parallel to one another.

The recessed inner faces of the first and second shoulder portions urge the double-disk device back toward the relaxed condition where the first and second shoulder portions are closest to one another. When deploying the device, the first and second shoulder portions are axially separated from one another, where the tension in the wire mesh of the inner faces is translated into a tension to bias the first and second disk portions back toward one another transmitted by the central waist portion. This structure allows the device to be flexed both axially and laterally such that it is capable of adjusting to the length and orientation of any given apical hole.

Further advantages of the present invention will become apparent from the discussion of preferred embodiments taken in conjunction with the drawings, which show:
Figure 1 shows a cross-sectional view of the occlusion device in the relaxed condition according to one embodiment of the invention;
Figure 2 shows a side view of the embodiment of Figure 1, where the wire mesh structure becomes visible;
Figure 3 shows an end view of the embodiment of Figures 1 and 2 taken along the center axis A of the device when looking in the direction from the smaller first disk to the larger second disk;
Figure 4 shows an embodiment by which the occlusion device is collapsed and located within the sheath of the delivery device;
Figures 5 and 6 show two stages of a deployment procedure of the occluder in an apical hole of the human heart.

### Description of Preferred Embodiments

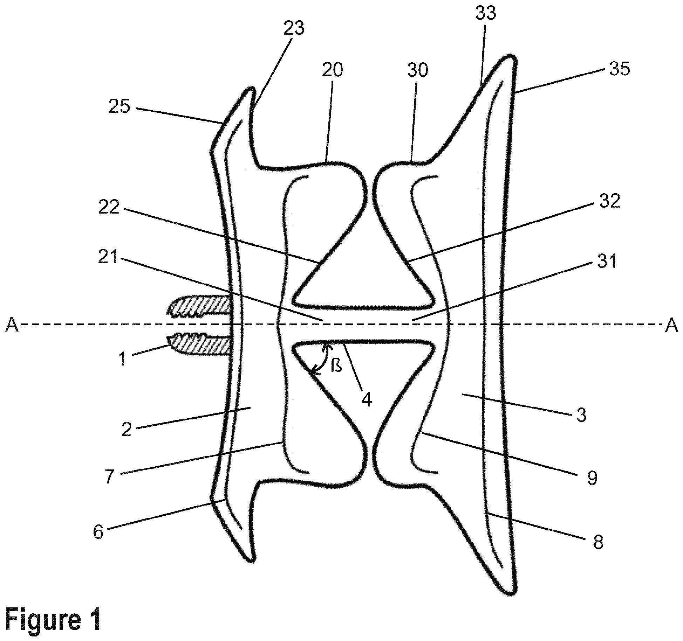

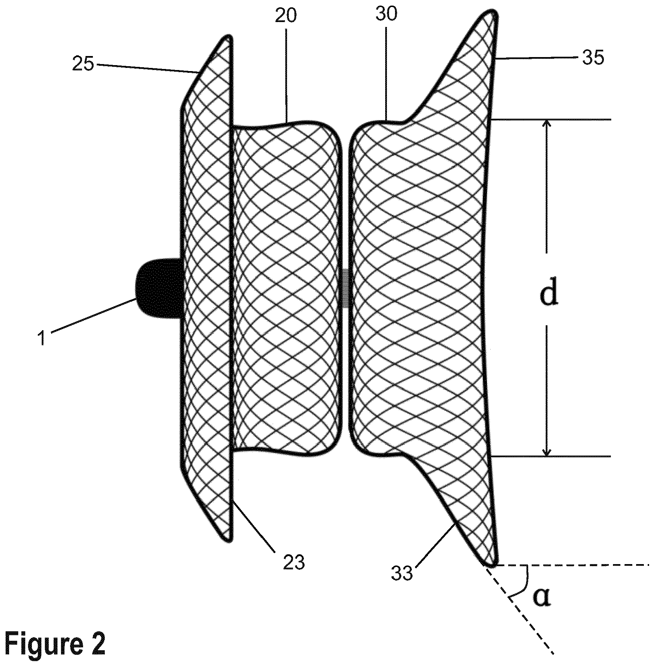

Figures 1 and 2 illustrate a first embodiment of the present invention, where the occlusion device is depicted in the relaxed condition, where the metal mesh components made of shape memory alloy wires are at their relaxed position. A first disk 2 having an enlarged diameter flange portion 25, shown on the left, is adapted to be placed proximate an exterior end of the apical hole in the patient's heart wall. The exterior end is the end of the hole facing the outside of the heart wall. The first disk 2 further comprises a first shoulder portion 20 which is adapted to be placed within the apical hole near the exterior end.

Likewise, a second disk 3 has an enlarged diameter flange portion 35 which is adapted to be placed proximate an interior end of the apical hole, i.e. the end which is within the patient's heart. A second shoulder portion 30 is adapted to be placed within the hole near the interior end. With this construction, the enlarged diameter portions can engage opposing surfaces of the heart wall adjacent the apical hole.

The occluder further comprises a central waist portion 4 which extends between the first and second disk portions 2, 3 along a center axis A of the device. The central waist portion 4 is adapted to extend through the apical hole and connect the first and second shoulder portions 20, 30 of the disks.

As can be taken from Figure 1, the first and second shoulder portions 20, 30 have respective inner faces 22, 32 which lie opposite one another in the direction of the center axis A. Each inner face 22, 32 is concaved inwardly within its corresponding shoulder portion 20, 30 by an angle β so as to form a recess in the relaxed condition as indicated. The angle β between the central waist 4 and the inner faces 22 and 32 may be adjusted so as to allow the axial extension of the occluder to increase or decrease to adapt to thickness of the heart wall.

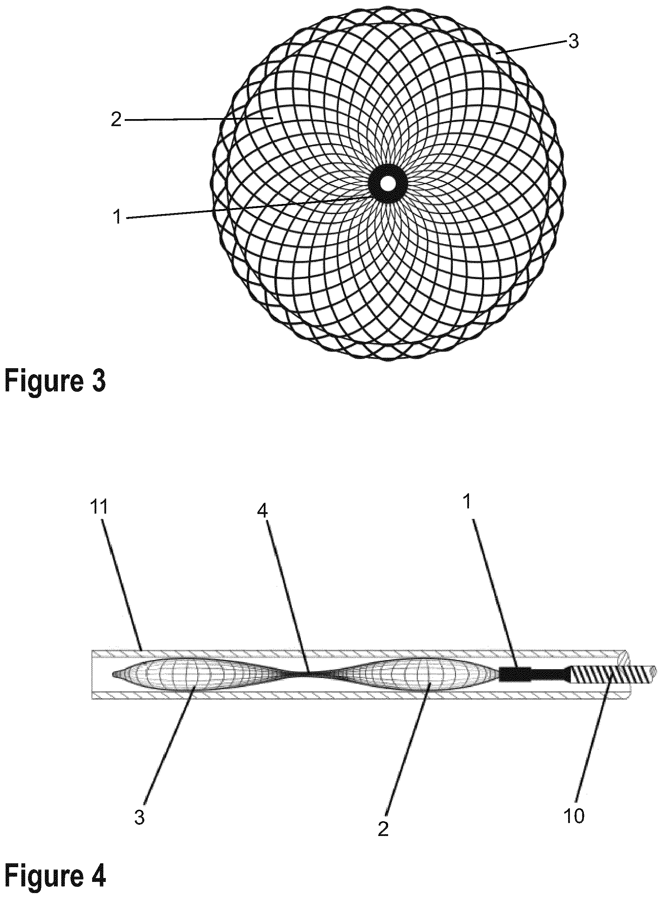

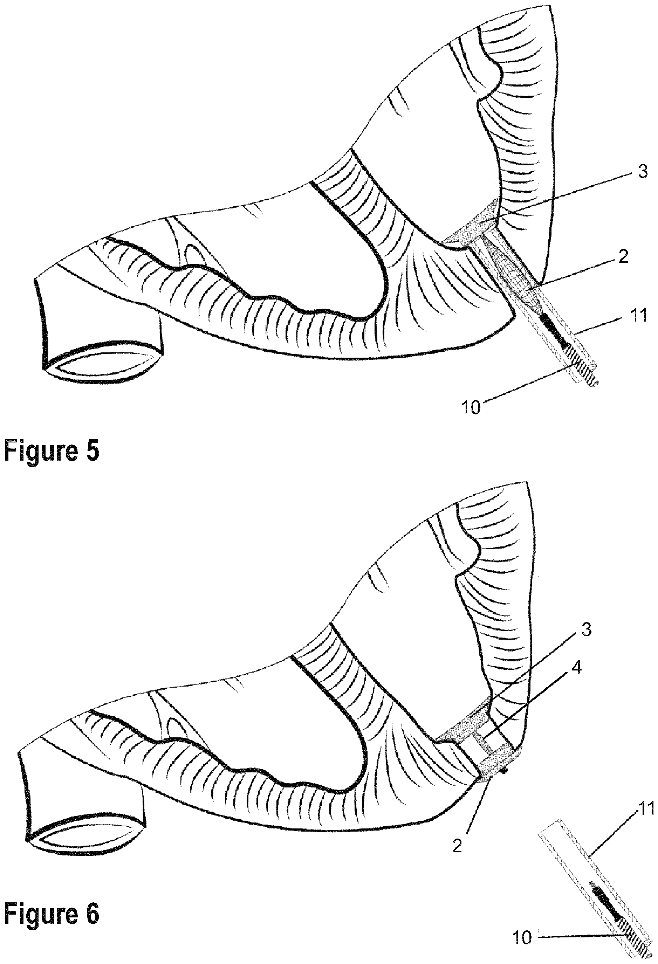

However, when the occluder is being deployed, the first and second disks are drawn apart from one another (see Figures 4 and 5 and the discussion below), such that the central waist portion 4 pulls the recessed inner faces 22, 32 from the relaxed condition shown in Figure 1 to a mounted condition shown in Figures 5 and 6 where the previously recessed inner faces now become substantially flat surfaces. With this arrangement, expanding the two disks to move apart axially during deployment is resisted by the mesh structure of the recessed inner faces 22, 32 of the shoulder portions. Or, starting from the expanded condition, the first and second shoulder portions 20, 30 are urged to return to the relaxed condition through the tensile force generated by the memory material of mesh structure of the inner faces 22, 32.

The central waist portion 4 comprises first and second side waist portions 21, 31 which are connected respectively to the inner faces 22, 32 of the first and second shoulder portions 20, 30 as shown in Figure 1. The central waist portion 4 may be formed integrally as one piece with the entire occluder, which is fabricated in a heat shaping process of the shape memory mesh material. The waist portion 4 is highly contracted in comparison to the outer disk portions of the occluder and will typically have a diameter of about 2 mm.

The overall length of the central waist portion 4 can be varied depending on the individual patient, but is at least 5 mm and generally in the range of 5 to 18 mm in length. Preferably the central waist portion is in the range of 5 to 12 mm.

The side waist portions 21, 31 are provided as a generally tubular mesh sections which join integrally with the mesh of the inner faces 22, 32. Preferably, each disk structure 2, 3 including the enlarged diameter flange portion 25, 35, the shoulder portion 20, 30 and the respective part 21 or 31 of the waist portion are all formed of a single piece of wire mesh in a heat treatment for shape memory production. The flange portions 25, 35 will generally have a diameter in the range of 10 to 18 mm. The shoulder portions have a diameter in the range of 5 to 10 mm.

The present design of the central waist portion in conjunction with the inner faces 22, 32 of the shoulder portions 20, 30 allow convenient adjustment of the length of the device. The construction allows a flexing of the opposing disks 2, 3 in both axial and lateral direction, so as to adjust to the length and orientation of the apical hole. At the same time, the disks are urged toward one another by tensioning arising from the mesh structure of the inner faces 22, 32.

As shown in Figures 1 and 2, an inner peripheral surface of the first enlarged diameter flange portion 25 is expanded to define a perimeter edge 23 which, when mounted, will contact an exterior surface of the heart wall adjacent the apical hole. Likewise, an inner peripheral surface of the second enlarged diameter flange portion 35 is expanded to define a perimeter edge 33 which, when mounted, will contact an interior surface of the heart wall adjacent the apical hole.

The perimeter edge 33 of the second flange portion 35 is shaped differently, so as to match the shape of the heart wall from within. As shown in Figures 5 and 6, the heart wall near the apical hole has an acute inclination angle with respect to the center axis A of the device. Preferably, the associated perimeter edge 33 will have a matching inclination angle α with respect to the center axis A of about 65 degrees to 80 degrees. A typical inclination angle will be about 70 degrees.

As illustrated in the embodiment of Figure 3, the diameter of the first disk 2 for placement at the exterior surface is somewhat smaller that the diameter of the second disk 3 for placement on the interior heart wall. As an example the first disk 2 can have a diameter of 12 mm and the second disk 3 a diameter of 14 mm. These dimensions facilitate a better fit of the occluder when deployed, which is adapted to the anatomy of the inside and outside heart wall.

In another preferred embodiment, the present occlusion device comprises at least one blood-flow inhibiting filter attached to each of the two disks 2, 3. As shown in Figure 1, the fabric filters 6, 8 can be placed adjacent to the surface of the enlarged diameter portions 25, 35. Alternatively, filter fabrics 7 and 9 can be placed adjacent to the inner faces 22, 32 of the respective shoulder portions 20, 30. Preferably, the blood flow inhibiting filter fabrics are secured by suturing the filter material to the wire mesh structure of the respective disks. In the preferred embodiment, each disk 2, 3 comprises two blood-flow restricting filters 6, 7 or 8, 9 attached to the respective disks 2 or 3.

In the present embodiment, the device comprises a clamp 1 adapted for releasable attachment to a delivery device, as shown for example in Figures 1 and 4. The clamp 1 can be provided with an internal or external threading for releasable attachment to the delivery device. Conventional devices provide clamps or connector portions on both ends of the double-disk occluder. The present design with only one clamp is patient friendly in terms of biocompatibility and can also aid endothelialization to a great extent. The collapsed configuration of the occluder as shown for example in Figure 4 is such that it can be delivered to an access site size of up to 34 Fr.

The placement of the present apical occlusion device is similar to that used in surgery for ventricle septal defect (VSD) and atrial septal defect (ASD) These are common and familiar procedures for the surgeon and requires much less time than the mentioned devices and procedures of the prior art. Another advantage is that the present occluder allows for adjustment to the length of the apical hole without harm to the myocardium tissue.

The deployment of the present occluder is accomplished with a delivery device comprising a sheath 11. A pusher device 10 can be screw-connected to the clamp 1 of the occluder (see Figure 4) and the sheath 11 is brought into the target area. The pusher device pushes the interior disk 3 out of the sheath as illustrated in Figure 5. The sheath is then further retracted until resistance is felt from the interior disk 3 when engaging the internal wall of the heart. The sheath 11 is then further retracted to deploy the second external disk 2 as shown in Figure 6. Finally, the threaded clamp 1 is unscrewed from the pusher and the entire delivery device with sheath 11 can be withdrawn.

The following is a clinical example of utilization of the present device in a TAVR procedure.

The left ventricular apical thickness of the patient's heart was computed from a tomographic scan to be 6.5 mm. During the procedure with a standard anterolateral minithoracotomy, two reinforced purse-string sutures were performed at the apex to prevent major bleeding in case of occluder failure.

The TA-TAVR procedure was performed, and a 23 mm Sapien XT valve was implanted. Following the procedure, the Ascendra delivery system was extracted and we inserted a 9 mm apex occluder under fluoroscopic guidance. The model occlude employed had a shoulder diameter of 7mm.

The distal part of the occluder was deployed in the LV and placed against the ventricular rim of the sheath 11. Then, we performed a pullback of both the sheath 11 and the model occluder until we experienced a certain degree of resistance, meaning that the internal part of the occluder touched the apex opening. At that time, and without rapid cardiac pacing, we retrieved only the sheath 11 and thereby immediately deployed the external part of the occluder 2 with a good result.

Only minor bleeding occurred that completely disappeared after protamine infusion. The occluder was still attached to the pusher 10 of the delivery system and still fully available in case of need. After 20 minutes of perfect functioning of the model occluder, it was disconnected from the delivery system and the purse-string sutures were tied only for safety reasons. Patient recovery was uneventful and without complications related to the occluder, and we restored anticoagulation treatment. After a 3-month follow-up period, the patients was asymptomatic and in good condition.

As illustrated above, the application process of present occluder for closing an apical puncture is completely sutureless, and is performed in an easy and rapid manner. Less blood loss results and the healing time is shortened. Based on the success with the use of proven occluder delivery and application systems, the utilization of the present occluder system to the cardiac apex is safe and reliable. The special design of the adjustable waist and the single clamp construction make the occluder unique, efficient and safe to use.

## Claims

1. An occlusion device, comprising:
a central waist portion (4) extending between first and second disks (2, 3) disposed along a center axis (A), the device being formed of metal mesh comprising a plurality of metal strands made of shape memory alloy material,
wherein the first disk (2) has a first enlarged diameter flange portion (25) adapted to be placed on an exterior surface of a patient's heart wall adjacent an exterior end of an apical hole in the heart wall,
and an adjacent first shoulder portion (20) of smaller diameter adapted to be placed within the apical hole near the exterior end,
wherein the second disk (3) has a second enlarged diameter portion (35) adapted to be placed on an interior surface of the heart wall adjacent the interior end of the apical hole,
and an adjacent second shoulder portion (30) of smaller diameter adapted to be placed within the apical hole near the interior end, and
wherein the central waist portion (4) is adapted to extend through the apical hole and connect the first and second disks (2, 3), while urging the disks (2, 3) toward one another when the device is in an expanded condition,
**characterized in that**
the first and second shoulder portions (20, 30) have respective inner faces (22, 32) lying opposite one another in the direction of the center axis (A), each inner face (22, 32) being concaved inwardly within its corresponding shoulder portion (20, 30) by an angle β between each of the inner faces (22, 32) and the central waist portion (4) so as to form a recess when the device is in a relaxed condition, and
when in the expanded condition of the mounted device within the apical hole, the disks (2, 3) are urged toward one another by the tensioning arising from the mesh structure of the inner faces (22, 32) of the first and second shoulder portions (20, 30) which take on a flatter shape as they are pulled toward one another by the central waist portion (4).

2. The occlusion device of claim 1, wherein the device can be placed into a collapsed configuration for delivery of the device into the apical hole of the patient through a lumen of a catheter.

3. The occlusion device of claim 1 or 2, wherein the central waist portion (4) comprises first and second side waist portions (21, 31) connected respectively to the inner faces (22, 32) of the first and second shoulder portions (20, 30) at positions along the center axis (A).

4. The occlusion device of claim 3, wherein the central waist portion (4) comprising the first and second side waist portions (21, 31) is formed integrally as one piece with the first and second disks.

5. The occlusion device of any one of the preceding claims, wherein the central waist portion (4) has a length of in the range of 5 mm to 18 mm.

6. The occlusion device of any one of the claims 1 to 5, wherein the inner faces (22, 23) of the shoulder portions (20, 30) and the central waist portion (4) are adapted to flex both axially and laterally such that the device is capable of adjusting to the length and orientation of the apical hole, while providing an inward biasing of the first and second disks (2, 3) toward one another.

7. The occlusion device of any one of the preceding claims, wherein a peripheral surface of the first enlarged diameter flange portion (25) is expanded to define an inner perimeter edge (23) which, when mounted, contacts an exterior surface of the heart wall adjacent the apical hole.

8. The occlusion device of any one of the preceding claims, wherein a peripheral surface of the second enlarged diameter flange portion (35) is expanded to define an inner perimeter edge (33) which, when mounted, contacts an interior surface of the heart wall adjacent the apical hole.

9. The occlusion device of claim 8, wherein the perimeter edge (33) for contact with an interior surface of the heart wall has an inclination angle (α) with respect to the center axis (A) of about 65 degrees to 80 degrees.

10. The occlusion device of any one of the preceding claims, wherein at least one blood flow inhibiting filter (6, 7; 8, 9) is provided in each of the first and second disks (2, 3).

11. The occlusion device of claim 10, wherein two blood flow inhibiting filters (6, 7; 8. 9) are provided in each disk (2, 3), one disposed adjacent the enlarged diameter flange portion (25, 35) and the other disposed adjacent an inner face (22, 32) of the shoulder portion (20, 30).

12. The occlusion device of any one of the preceding claims, wherein the first disk (2) includes a clamp (1) adapted for releasable attachment to a delivery device (10), in particular a threaded clamp (1).

## Patentansprüche

1. Okklusionsvorrichtung mit:
einem mittleren verengten Abschnitt (4), der sich zwischen einer ersten und einer zweiten Scheibe (2, 3) erstreckt, die längs einer Mittelachse (A) angeordnet sind, wobei die Vorrichtung aus einem Metallgeflecht gebildet ist, das mehrere Metallfäden aus einem Formgedächtnislegierungsmaterial umfasst,
wobei die erste Scheibe (2) einen ersten Flanschabschnitt (25) mit vergrößertem Durchmesser aufweist, der zur Anordnung auf einer Außenfläche der Herzwand eines Patienten angrenzend an ein äußeres Ende eines apikalen Lochs in der Herzwand ausgebildet ist,
und einem angrenzenden ersten Schulterabschnitt (20) mit kleinerem Durchmesser, der zur Anordnung in dem apikalen Loch nahe dem äußeren Ende ausgebildet ist,
wobei die zweite Scheibe (3) einen zweiten Abschnitt (35) mit vergrößertem Durchmesser aufweist, der zur Anordnung auf einer Innenfläche der Herzwand angrenzend an das innere Ende des apikalen Lochs ausgebildet ist,
und einem angrenzenden zweiten Schulterabschnitt (30) mit kleinerem Durchmesser, der zur Anordnung in dem apikalen Loch nahe dem inneren Ende ausgebildet ist, und
wobei der mittlere verengte Abschnitt (4) so ausgebildet ist, dass er sich durch das apikale Loch erstreckt und die erste und die zweite Scheibe (2, 3) verbindet, während er die Scheiben (2, 3) aufeinander zu vorspannt, wenn sich die Vorrichtung in einem expandierten Zustand befindet,
**dadurch gekennzeichnet, dass**
der erste und der zweite Schulterabschnitt (20, 30) jeweilige Innenflächen (22, 32) aufweisen, die in Richtung der Mittelachse (A) einander gegenüberliegen, wobei jede Innenfläche (22, 32) innerhalb ihres entsprechenden Schulterabschnitts (20, 30) mit einen Winkel β zwischen jeder der Innenflächen (22, 32) und dem mittleren verengten Abschnitt (4) nach innen konkav ausgebildet ist, derart, dass eine Ausnehmung gebildet ist, wenn sich die Vorrichtung in einem entspannten Zustand befindet, und
dann, wenn sich die in dem apikalen Loch angebrachte Vorrichtung im expandierten Zustand befindet, durch das Spannen, das sich aus der Geflechtstruktur der Innenflächen (22, 32) des ersten und des zweiten Schulterabschnitts (20, 30) ergibt, die eine flachere Form annehmen, während sie vom mittleren verengten Abschnitt (4) aufeinander zu gezogen werden, die Scheiben (2, 3) aufeinander zu vorgespannt sind.

2. Okklusionsvorrichtung nach Anspruch 1, wobei die Vorrichtung in eine zusammengeklappte Gestalt gebracht werden kann, um die Vorrichtung durch ein Lumen eines Katheters in das apikale Loch des Patienten einzubringen.

3. Okklusionsvorrichtung nach Anspruch 1 oder 2, wobei der mittlere verengte Abschnitt (4) einen ersten und einen zweiten seitlichen verengten Abschnitt (21, 31) umfasst, die an Positionen längs der Mittelachse (A) mit der jeweiligen Innenfläche (22, 32) des ersten bzw. des zweiten Schulterabschnitts (20, 30) verbunden sind.

4. Okklusionsvorrichtung nach Anspruch 3, wobei der mittlere verengte Abschnitt (4), der den ersten und den zweiten seitlichen verengten Abschnitt (21, 31) umfasst, als ein Teil einstückig mit der ersten und der zweiten Scheibe ausgebildet ist.

5. Okklusionsvorrichtung nach einem der vorhergehenden Ansprüche, wobei der mittlere verengte Abschnitt (4) eine Länge im Bereich von 5 mm bis 18 mm aufweist.

6. Okklusionsvorrichtung nach einem der Ansprüche 1 bis 5, wobei die Innenflächen (22, 23) der Schulterabschnitte (20, 30) und der mittlere verengte Abschnitt (4) so ausgebildet sind, dass sie sich sowohl axial als auch lateral so biegen, dass die Vorrichtung in der Lage ist, sich an die Länge und Ausrichtung des apikalen Lochs anzupassen, während sie eine Beaufschlagung der ersten und der zweiten Scheibe (2, 3) nach innen aufeinander zu bereitstellt.

7. Okklusionsvorrichtung nach einem der vorhergehenden Ansprüche, wobei eine Umfangsfläche des ersten Flanschabschnitts (25) mit vergrößertem Durchmesser so erweitert ist, dass sie einen inneren Umfassungsrand (23) bildet, der im angebrachten Zustand mit einer Außenfläche der Herzwand angrenzend an das apikale Loch in Anlage ist.

8. Okklusionsvorrichtung nach einem der vorhergehenden Ansprüche, wobei eine Umfangsfläche des zweiten Flanschabschnitts (35) mit vergrößertem Durchmesser so erweitert ist, dass sie einen inneren Umfassungsrand (33) bildet, der im angebrachten Zustand mit einer Innenfläche der Herzwand angrenzend an das apikale Loch in Anlage ist.

9. Okklusionsvorrichtung nach Anspruch 8, wobei der Umfassungsrand (33) zur Anlage an einer Innenfläche der Herzwand einen Neigungswinkel (α) von etwa 65 Grad bis 80 Grad zur Mittelachse (A) aufweist.

10. Okklusionsvorrichtung nach einem der vorhergehenden Ansprüche, wobei in der ersten und der zweiten Scheibe (2, 3) jeweils wenigstens ein blutflusshemmender Filter (6, 7; 8, 9) vorgesehen ist.

11. Okklusionsvorrichtung nach Anspruch 10, wobei in jeder Scheibe (2, 3) zwei blutflusshemmende Filter (6, 7; 8, 9) vorgesehen sind, von denen einer angrenzend an den Flanschabschnitt (25, 35) mit vergrößertem Durchmesser und der andere angrenzend an eine Innenfläche (22, 32) des Schulterabschnitts (20, 30) angeordnet ist.

12. Okklusionsvorrichtung nach einem der vorhergehenden Ansprüche, wobei die erste Scheibe (2) eine Klammer (1) aufweist, die zur lösbaren Befestigung an einer Einbringungsvorrichtung (10) ausgebildet ist, insbesondere eine Gewindeklammer (1).

## Revendications

1. Dispositif d'occlusion, comprenant :
un tronçon d'étranglement médian (4) qui s'étend entre un premier et un deuxième disque (2, 3) qui sont agencés le long d'un axe médian (A), le dispositif étant formé par un treillis métallique comprenant une pluralité de fils métalliques réalisés en un matériau d'alliage à mémoire de forme,
le premier disque (2) présentant un premier tronçon de collerette (25) de diamètre agrandi qui est apte à être placé sur une surface extérieure d'une paroi de coeur d'un patient de manière adjacente à une extrémité extérieure d'un trou apical dans la paroi de coeur,
et un premier tronçon d'épaulement (20) adjacent de diamètre inférieur qui est apte à être placé à l'intérieur du trou apical près de l'extrémité extérieure,
le deuxième disque (3) présentant un deuxième tronçon (35) de diamètre agrandi qui est apte à être placé sur une surface intérieure de la paroi de coeur de manière adjacente à l'extrémité intérieure du trou apical,
et un deuxième tronçon d'épaulement (30) adjacent de diamètre inférieur qui est apte à être placé à l'intérieur du trou apical près de l'extrémité intérieure, et
le tronçon d'étranglement médian (4) étant apte à s'étendre à travers le trou apical et à relier le premier et le deuxième disque (2, 3) en sollicitant les disques (2, 3) l'un vers l'autre lorsque le dispositif se trouve dans un état évasé,
**caractérisé en ce que**
le premier et le deuxième tronçon d'épaulement (20, 30) présentent des faces intérieures respectives (22, 32) qui sont opposées l'une à l'autre dans le sens de l'axe médian (A), chaque face intérieure (22, 32) étant concave vers l'intérieur au sein de son tronçon d'épaulement correspondant (20, 30) selon un angle β entre chacune des faces intérieures (22, 32) et le tronçon d'étranglement médian (4), de manière à former un évidement lorsque le dispositif se trouve dans un état détendu, et
**en ce qu'**à l'état évasé du dispositif installé au sein du trou apical, les disques (2, 3) sont sollicités l'un vers l'autre par la tension résultant de la structure de treillis des faces intérieures (22, 32) du premier et du deuxième tronçon d'épaulement (20, 30) qui prennent une forme plus plate lorsqu'ils sont tirés l'un vers l'autre par le tronçon d'étranglement médian (4).

2. Dispositif d'occlusion selon la revendication 1, le dispositif étant apte à être placé selon une configuration repliée pour amener le dispositif dans le trou apical du patient à travers une cavité d'un cathéter.

3. Dispositif d'occlusion selon la revendication 1 ou 2, le tronçon d'étranglement médian (4) comportant un premier et un deuxième tronçon d'étranglement latéral (21, 31) qui sont reliés aux faces intérieures (22, 32) du premier et du deuxième tronçon d'épaulement (20, 30), respectivement, à des endroits le long de l'axe médian (A).

4. Dispositif d'occlusion selon la revendication 3, le tronçon d'étranglement médian (4) comportant le premier et le deuxième tronçon d'étranglement latéral (21, 31) étant intégralement réalisé d'une seule pièce avec le premier et le deuxième disque.

5. Dispositif d'occlusion selon l'une des revendications précédentes, le tronçon d'étranglement médian (4) présentant une longueur dans la plage de 5 mm à 18 mm.

6. Dispositif d'occlusion selon l'une des revendications 1 à 5, les faces intérieures (22, 23) des tronçons d'épaulement (20, 30) et le tronçon d'étranglement médian (4) étant aptes à fléchir axialement et latéralement de sorte que le dispositif est apte à s'adapter à la longueur et à l'orientation du trou apical en fournissant une sollicitation du premier et du deuxième disque (2, 3) l'un vers l'autre vers l'intérieur.

7. Dispositif d'occlusion selon l'une des revendications précédentes, une surface périphérique du premier tronçon de collerette (25) de diamètre agrandi étant évasée de manière à définir un bord de périmètre intérieur (23) qui, à l'état installé, est en contact avec une surface extérieure de la paroi de coeur adjacente au trou apical.

8. Dispositif d'occlusion selon l'une des revendications précédentes, une surface périphérique du deuxième tronçon de collerette (35) de diamètre agrandi étant évasée de manière à définir un bord de périmètre intérieur (33) qui, à l'état installé, est en contact avec une surface intérieure de la paroi de coeur adjacente au trou apical.

9. Dispositif d'occlusion selon la revendication 8, le bord de périmètre (33) pour le contact avec une surface intérieure de la paroi de coeur présentant par rapport à l'axe médian (A) un angle d'inclinaison (α) d'environ 65 degrés à 80 degrés.

10. Dispositif d'occlusion selon l'une des revendications précédentes, au moins un filtre inhibiteur de flux sanguin (6, 7 ; 8, 9) étant prévu dans le premier et dans le deuxième disque (2, 3).

11. Dispositif d'occlusion selon la revendication 10, deux filtres inhibiteurs de flux sanguin (6, 7 ; 8, 9) étant prévus dans chaque disque (2, 3), l'un étant agencé adjacent au tronçon de collerette (25, 35) de diamètre agrandi, et l'autre étant agencé adjacent à une face intérieure (22, 32) du tronçon d'épaulement (20, 30).

12. Dispositif d'occlusion selon l'une des revendications précédentes, le premier disque (2) présentant une attache (1) adaptée à une fixation détachable sur un dispositif d'amenée (10), en particulier une attache filetée (1).
